# EUROPEAN PATENT SPECIFICATION

(11) **EP 0 738 253 B1**
(45) Date of publication and mention of the grant of the patent: **05.09.2001**
(21) Application number: 94909428.8
(22) Date of filing: 03.12.1993
(51) Int. Cl.: C07C 59/21, C07C 69/66, C07C 59/42

(54) **CARBONYL CONTAINING COMPOUNDS**
CARBONYLGRUPPEN ENTHALTENDE VERBINDUNGEN
COMPOSES RENFERMANT UNE FONCTION CARBONYLE

(43) Date of publication of application: 23.10.1996
(73) Proprietor: Infineum USA L.P., Linden, New Jersey 07036 (US)
(72) Inventor: BROIS, Stanley, J., Westfield, NJ 07090 (US)
(74) Representative: Bawden, Peter Charles
(86) International application number: US9312707
(87) International publication number: WO9515303

(56) References cited:
- US-A- 4 566 984
- US-A- 5 057 564
- US-A- 5 288 811
- HETEROCYCLES, Vol. 27, No. 9, "Thermal Reactions of Donor-Acceptor Systems...", (MATTAY et al.), pages 2156-2165, 1988.
- J. Am. Chem. Soc. 1984, 106, 3797-802
- J. Org. Chem. 1980, 45, 4774-6
- J. Org. Chem. 1980, 45, 1228-39

## Description

The present invention relates to novel compounds formed from acyclic carbonyl compounds and unsaturated hydrocarbons.

### BACKGROUND OF THE INVENTION

Various unsaturated hydrocarbon polymers have been reacted with maleic anhydrides to form a variety of maleic anhydride adducts of unsaturated hydrocarbon polymers. The reactivity of maleic anhydride with many unsaturated hydrocarbon polymers is poor and in some instances, as for example with EPDM rubber, even employment of extensive heating is ineffective. Free employment of extensive heating is ineffective. Free radical reactions which graft maleic anhydride onto the unsaturated hydrocarbon polymer have been utilized as alternative routes. Free radical grafting leads to chain scission, crosslinking and solvent grafting if the solvent is sufficiently reactive. The reaction of acyclic carbonyl monomers with the unsaturated hydrocarbon polymer overcomes these aforementioned deficiencies in that the acyclic carbonyl monomers can be reacted with the unsaturated hydrocarbon polymer at moderate temperature, in either the bulk or solution state without the employment of free radical initiators to form novel polymers which are useful as solution viscosifiers.

### SUMMARY OF THE INVENTION

In accordance with the present invention, there is provided a novel composition of matter having the formula: 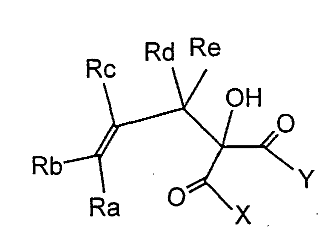 wherein that portion of the formula including Ra, Rb, Rc, Rd and Re has a molecular weight of from 500 to 10⁷,
wherein Rₐ, R_{b}, R_{c}, Rd and Rₑ are independently selected from the group consisting of H, alkyl groups and substituted alkyl groups having 1 to 10⁶ carbon atoms, alkenyl groups and substituted alkenyl groups having 3 to 10⁶ carbon atoms, wherein the substituents on the alkyl and/or alkenyl groups are selected from the group consisting of alkoxy, halogen, CN, OH, HO(CH₂CH₂0)ₓ (X=1-10), acyl, acyloxy, aryl substituents and 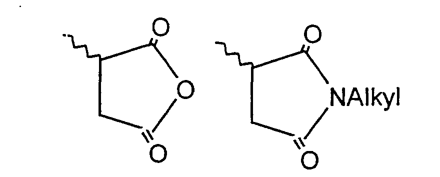 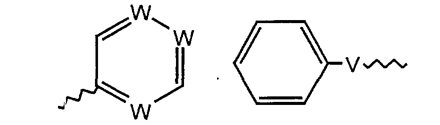 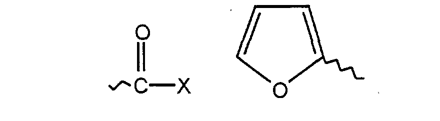 wherein W=C, N; V=O, S, SO₂; and X is selected from the group consisting of -OH; -OR₁, -NR₁R₂; -R₁; wherein R₁ is a group having from 1 to 18 carbon atoms and wherein R₂ is hydrogen or any alkyl group having from 1 to 18 carbon atoms.

These novel compounds are formed by contacting an olefinic polymer containing an allylic hydrogen and having a molecular weight of from 500 to 10⁷, selected from polymers having the formula: 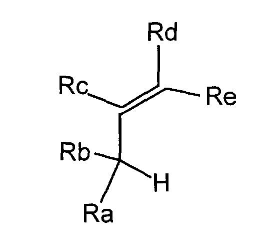 or an EPDM terpolymer containing ethylene, propylene and a non-conjugated diene, with an acyclic carbonyl having the formula: 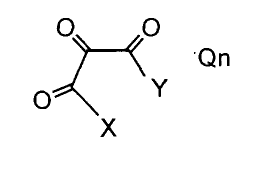 for a time and at a temperature sufficient to form the compounds, and in which Rₐ, R_{b}, R_{c}, R_{d} and Rₑ are as described above and Q = HOH, MeOH, EtOH, or n-BuOH; n=0,1, >1; X or Y are selected from the group consisting of -OH; -OR₁; NR₁R₂; R₁; and phenyl wherein R₁ is a group having 1 to 18 carbon atoms, wherein R₂ is hydrogen or any alkyl group of from 1 to 18 carbon atoms. Typical monomers are ketomalonic acid, esters of ketomalonic acid including alkyl and aryl esters; other useful ketoacids are alpha keto succinic acid, diketo succinic acid, and any alpha ketohydrocarboic acid and alpha, betadiketohydrocarboic acids and their ester and amide analogs which have a molecular weight of 130 to 500. Useful ketones include dimethyl, diphenyl and di-tolyl tri- and tetraketones.

The compounds of the present invention are useful as solution viscosification agents.

### GENERAL DESCRIPTION

Compounds having the formula: 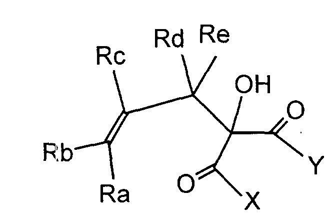 are prepared by contacting an olefinic compound and an acyclic carbonyl compound for a time and at a temperature sufficient to form the compound. Thus, a typical reaction to produce these novel carbonyl compounds is represented by the equation: 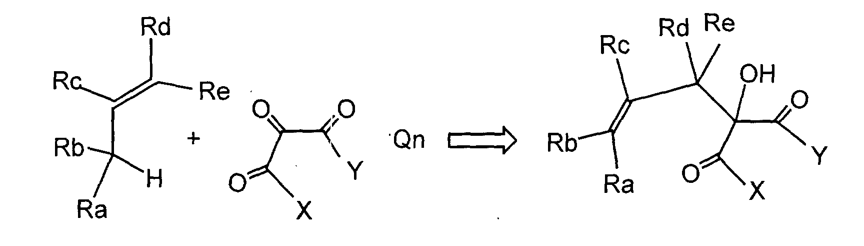 wherein Rₐ, R_{b}, R_{c}, R_{d} and Rₑ are independently selected from the group consisting of H, alkyl groups and substituted alkyl groups having 1 to 10⁶ carbon atoms, alkenyl groups and substituted alkenyl groups having 3 to 10⁶ carbon atoms, wherein the substituents on the alkyl and/or alkenyl groups are selected from the group consisting of alkoxy, halogen, CN, OH, HO(CH₂CH₂0)x (x=1-10), acyl, acyloxy and aryl substituents, and 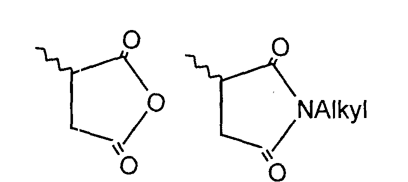 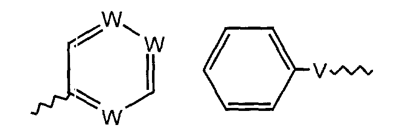 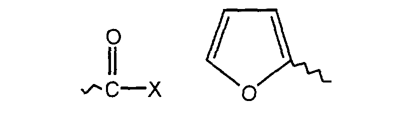 wherein W=C, N; V=O, S, SO₂; and X is selected from the group consisting of OH; -OR₁, NR₁R₂; R₁; wherein R₁ is a group having from 1 to 18 carbon atoms, and wherein R₂ is hydrogen or any alkyl and having from 1 to 18 carbon atoms, Q = HOH, MeOH, EtoH, or n-BuOH; n=0,1,>1; X or Y are selected from the group consisting of -OH; -OR₁; NR₁R₂; R₁; and phenyl wherein R₁ is from 1 to 18 carbon atoms, wherein R₂ is hydrogen or any alkyl group of from 1 to 18 carbon atoms. Typical monomers are ketomalonic acid, esters of ketomalonic acid including alkyl and aryl esters; other useful ketoacids are alpha keto succinic acid, diketo succinic acid, and any alpha ketohydrocarboic acid and alpha, betadiketohydrocarboic acids and their ester and amide analogs which have a molecular weight of about 130 to 500. Useful ketones include dimethyl, diphenyl and di-tolyl tri- and tetraketones.

The olefinic hydrocarbons are olefinic polymers containing an allylic hydrogen and having molecular weights ranging from 500 to 10,000,000. The olefinic hydrocarbons may, of course, be substituted with functionalities such as -CN, -OH, HO(CH₂CH₂0)x (x=1-10), alkoxy, halogen, and 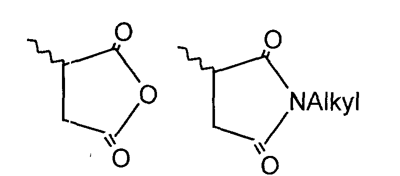 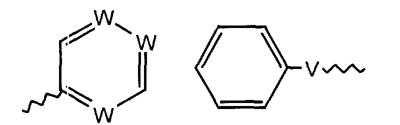 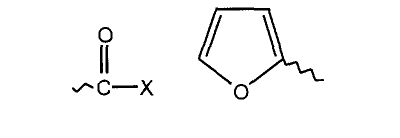 wherein W=C, N; V=O, S, SO₂; and x is selected from the group consisting of -OH; -OR₁, -NR₁R₂; -R₁; wherein R₁ has 1 to 18 carbon atoms, wherein R₂ is hydrogen or any alkyl and has 1 to 18 carbon atoms. Typical substituted alkenes include polyisobutenyl succinic anhydride, and related functional olefins and polyolefins.

Among the preferred polymers are butyl rubber and EPDM polymers. The expression "butyl rubber" as employed in the specification and claims is intended to include copolymers made from a polymerization reaction mixture having therein from 70 to 99.5% by weight of an isobutylene and 0.5 to 30% by weight of a conjugated multiolefin having from 4 to 14 carbon atoms, e.g., isoprene. The resulting copolymer contains 85 to 99.8% by weight of combined isoolefin and 0.2 to 15% of combined multiolefin.

Butyl rubber generally has a Staudinger molecular weight as measured by GPC of 20,000 to 500,000, preferably 25,000 to 400,000, especially 100,000 to 400,000 and a Wijs Iodine No. of 0.5 to 50, preferably 1 to 15. The preparation of butyl rubber is described in U.S. Patent No. 2,356,128.

For the purposes of this invention, the butyl rubber may have incorporated therein from 0.2 to 10% of combined multiolefin; preferably 0.5 to 6%, more preferably 1 to 4%, e.g., 2%.

Illustrative of such a butyl rubber is Exxon butyl 365 (Exxon Chemical Company), having a mole percent unsaturation of 2.0% and a Mooney viscosity (ML, 1+3, 212°F) of 40 to 50.

Low molecular weight butyl rubbers, i.e., butyl rubbers having a viscosity average molecular weight of 5,000 to 85,000, and a mole percent unsaturation of 1 to 5%. Preferably, these polymers have a viscosity average molecular weight of 25,000 to 60,000.

The EPDM terpolymers are low unsaturated polymers having 0.5 to 10.0 wt% olefinic unsaturation, more preferably 2 to 8, most preferably 3 to 7 defined according to the definition as found in ASTM-1418-64 and is intended to mean terpolymers containing ethylene and propylene in the backbone and an olefin residue in the side chain as a result of multiolefin incorporation in the backbone. Illustrative methods for producing these terpolymers are found in U.S. Patent No. 3,280,082, British Patent No. 1,030,289 and French Patent No. 1,386,600. The preferred polymers contain 40 to 75 wt% ethylene and 1 to 10 wt% of a diene monomer, the balance of the polymer being propylene. Preferably, the polymer contains 45 to 70 wt% ethylene, e.g., 50 wt% and 2.6 to 8.0 wt% diene monomer, e.g., 5.0 wt%. The diene monomer is a nonconjugated diene.

Illustrative of these nonconjugated diene monomers which may be used in the terpolymer (EPDM) are 1,4-hexadiene, dicyclopentadiene, 4-ethylidene-2-norbornene, 5-methylene-2-norbornene, 5-propenyl-norbornene, methyl tetrahydroindene and 4-methyl-methylene-2-norbornene.

A typical EPDM is Vistalon 2504 (sold by Exxon Chemical Company, Houston, Texas), a terpolymer having a Mooney viscosity (ML, 1+8, 212°F) of 40 and having an ethylene content of 50 wt% and a ethylidene-2-norbornene content of 5.0 wt%. The Mₙ as measured by GPC of Vistalon 2504 is 47,000, the Mᵥ as measured by GPC is 145,000 and the M_{w} as measured by GPC is 174,000.

Another EPDM terpolymer Vistalon 2504-20 is derived from Vistalon 2504 (also sold by Exxon Chemical Company) by a controlled extrusion process, wherein the resultant Mooney viscosity at 212°F is 20. The Mₙ as measured by GPC of Vistalon 2504-20 is 26,000, the M_{y} as measured by GPC is 90,000 and the M_{w} as measured by GPC is 125,000.

Nordel 1320 (sold by Dupont, Wilrnington, Delaware) is another terpolymer having a Mooney viscosity at 212°F of 25 and having 53 wt% of ethylene, 3.5 wt% of 1,4-hexadiene, and 43.5 wt% of propylene.

The EPDM terpolymers of this invention have a number average molecular weight (Mₙ) as measured by GPC of 10,000 to 200,000, more preferably of 15,000 to 100,000, most preferably of 20,000 to 60,000. The Mooney viscosity (ML, 1+8, 212°F) of the EPDM terpolymer is 5 to 60, more preferably 10 to 50, most preferably 15 to 40. The Mᵥ as measured by GPC of the EPDM terpolymer is preferably below 350,000 and more preferably below 300,000. The M_{w} as measured by GPC of the EPDM terpolymer is preferably below 500,000 and more preferably below 350,000.

Other suitable olefin polymers having Mₙ of 500 to 10⁶ include polymers comprising a major molar amount of C₂ to C₅ monoolefins, e.g., ethylene, propylene, butylene, isobutylene and pentene. The polymers may be homopolymers such as polyisobutylene, as well as copolymers of two or more such olefins such as copolymers of ethylene and propylene, butylene and isobutylene, propylene and isobutylene.

The reaction of the acyclic carbonyl compound with the olefinic containing compound can occur in solution, in a melt and in polymer processing equipment such as a rubber mill, a Brabender, an extruder or a Banbury mixer.

Ene adductions can also be effected with acid catalysts such as kaolin, montmorillonite, silicates, SnCl₄, FeCl₃, and BF₃, which facilitate adduct formation. Moreover, the acid catalysts can produce lactones, secondary ene adducts and cyclic ethers, the product ratios varying with reaction conditions, and catalyst and reactant types.

The time and temperature for contacting can be varied widely and will depend, in part, on whether a catalyst is present. In general, the acyclic carbonyl compound is contacted with the olefinic containing compound in solution at temperatures ranging from 50°C to 220°C for times ranging from 4 to 40 hours.

Typically, the olefinic compound is dissolved in a suitable solvent, such as tetrahydrofuran, xylene or mineral oil and heated to temperatures ranging from 50°C to 220°C. The carbonyl compound, as a hydrate or hemiketal of methanol, butanol, or a suitable alcohol, is dissolved in a suitable solvent such as tetrahydrofuran, dioxane, or butanol, and added to the heated olefin solution. The reaction mixture is heated, with stirring, until infrared and NMR analysis of the mixture indicates that the ene-addition of the carbonyl monomer to the unsaturated polymer is complete. Depending on temperature and concentration, reaction periods of 4 to 40 hours are sufficient to achieve high conversions to mono-and/or multiple ene adducts.

Optionally, bulk reactions can be carried out at 80°C to 200°C for 3 to 300 minutes, depending upon the polyolefin used, the carbonyl compound reactivity, and use of a catalyst.

If necessary, products can be isolated by solvent removal by evaporation, or by adding the reaction mixture to a polar solvent such as acetone, which induces the precipitation of the functionalized polymer.

### DESCRIPTION OF THE PREFERRED EMBODIMENTS

The following examples illustrate the present invention without, however, limiting the same hereto.

### Example 1

One hundred grams of polyisobutylene, MW 950, and 34.0 grams of diethyl ketomalonate were combined in a reaction flask, stirred magnetically, and heated at 200°C for 40 hours. Rotoevaporation of the reaction mixture at 100°C for 8 hours afforded a residue which featured (a) an infrared spectrum with a strong ester carbonyl absorption band at 5.85 microns, and (b) a saponification number of 92.

### Example 2

One hundred grams of polyisobutylene succinic anhydride (MW-1050) having a saponification number of 55 were combined with 17 grams of diethyl ketomalonate, and heated to 200°C for 48 hours.

Rotoevaporation of the reaction mixture at 100°C for 8 hours gave a residue which featured an infrared spectrum having anhydride and ester carbonyl absorption bands at 5.65 and 5.85 microns, respectively.

### Example 3

A mixture of 95 grams of polyisobutylene (MW - 950), 10 grams of malefic anhydride, and 17.4 grams of diethyl ketomalonate was heated at 210°C for 40 hours. The cooled reaction mixture was dissolved in 500 ml of cyclohexane, filtered and rotoevaporated at 100°C for 8 hours. The residue featured an infrared spectrum with strong anhydride, and ester carbonyl absorption bands at 5.65 and 5.85 microns, respectively.

### Example 4

Ten grams of Vistalon-7504, an ethylidene norbornene (ENB) terpolymer containing about 52% ethylene, 43% propylene and 5% ENB, and having a Mn - 55,000, were dissolved in 100 ml of xylene containing 4 grams of diethyl ketomalonate. The mixture was heated to 135°C and maintained at 135°C for 30 hours under a blanket of nitrogen. Addition of the cooled reaction mixture to one liter of acetone caused the functionalized polymer to precipitate from solution. The dried polymer analyzed for 5.04% oxygen, and featured an infrared spectrum (film) with an intense ester carbonyl band at 5.82 microns, consistent with ene adducts including structure (B) shown below:

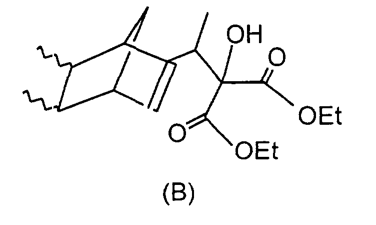

## Claims

1. A compound formed by reacting an olefinic polymer containing an allylic hydrogen and having a molecular weight of from 500 to 10⁷, selected from polymers having the formula: 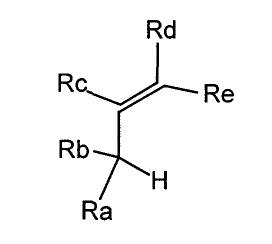 or an EPDM terpolymer containing ethylene, propylene and a non-conjugated diene monomer wherein Ra, Rb, Rc, Rd, Re are independently selected from H, alkyl groups and substituted alkyl groups having 1 to 10⁶ carbon atoms, alkenyl groups and substituted alkenyl groups having 3 to 10⁶ carbon atoms, wherein the substituents are selected from alkoxy, halogen, CN, OH, HO(CH2CH2O)x where x is from 1 to 10, acyl, acyloxy, aryl substituents and 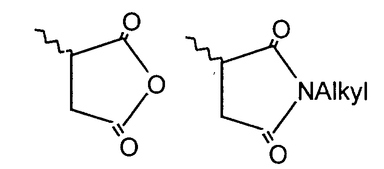 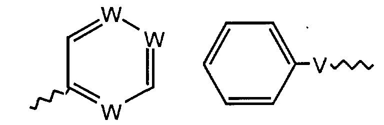 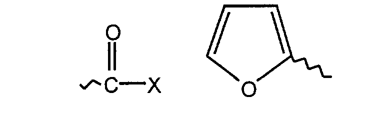 wherein W=C, N; V=O, S, SO₂; and X is selected from the group consisting of -OH; -OR₁; -NR₁R₂; -R₁; wherein R₁ is a group having from 1 to 18 carbon atoms, and wherein R₂ is hydrogen or any alkyl and having from 1 to 18 carbon atoms,
with an acyclic compound selected from
(A) an acyclic carbonyl having the formula: 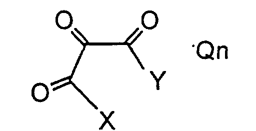 where Q=HOH, MeOH, EtOH, or n-BuOH; n=0, 1,>1, and X and Y are independently selected from -OH, -OR₁, -NR₁R₂,-R₁, and phenyl wherein R₁ is a group having from 1 to 18 carbon atoms, and R₂ is hydrogen or an alkyl group having from 1 to 18 carbon atoms and
(B) alpha keto succinic acid, diketosuccinic acid, alpha keto hydrocarboic acids and alpha, beta diketo hydrocarboic acids, and the ester and amide analogs of the alpha keto and alpha, beta diketo hydrocarboic acids, with the proviso that the molecular weight of the alpha keto and alpha beta diketo carboic acids, and their ester and amide analogs is between 130 and 500.

2. The compound of claim 1 wherein the compound is produced by reaction in a solvent at temperatures of from 50°C to 220°C.

3. The compound of either of claims 1 or 2 wherein the compound is produced by reaction in bulk at temperatures from 80°C to 220°C for from 3 to 300 minutes.

4. The compounds of either of claim 2 or 3 wherein the compound is produced by reaction in the presence of an acid catalyst.

5. A compound having the formula: 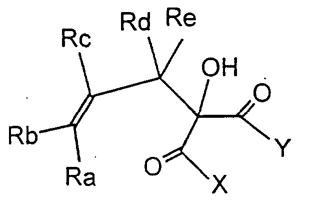 wherein Ra, Rb, Rc, Rd and Re are independently selected from H, alkyl groups and substituted alkyl groups having from 1 to 10⁶ carbon atoms, alkenyl groups and substituted alkenyl groups having from 3 to 10⁶ carbon atoms, wherein the substitutents on the alkyl and/or alkenyl groups are selected from alkoxy, halogen, CN, OH, HO(CH₂CH₂O)x (where x is 1 to 10), acyl, acyloxy aryl groups and 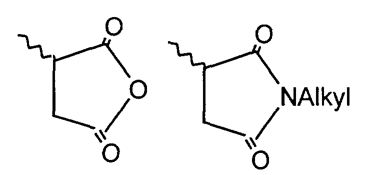 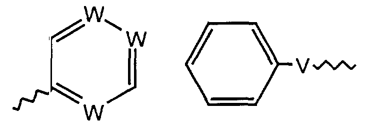 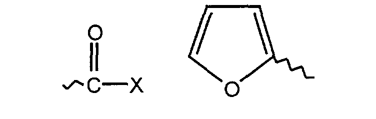 wherein W=C, N; V=O, S, SO₂; and X is selected from the group consisting of -OH; -OR₁, -NR₁R₂; R₁; wherein R₁ is a group having from 1 to 18 carbon atoms, and wherein R₂ is hydrogen or any alkyl group having from 1 to 18 carbon atoms,
X and Y are independently selected from -OH, -OR₁, -NR₁R₂,-R₁ and phenyl where R₁ is a group having from 1 to 18 carbon atoms and R₂ is H or an alkyl group having from 1 to 18 atoms
and wherein that portion of the formula including Ra, Rb, Rc, Rd and Re has a molecular weight of from 500 to 10⁷.

6. A process for producing a carbonyl containing compound according to claim 1 or 5 comprising reacting the unsaturated hydrocarbon polymer containing allylic hydrogen having a molecular weight of from 500 to 107 with the acyclic carbonyl compound in the absence of free radical initiators.

7. The process of claim 6 wherein the process is conducted in solution at temperatures from 50°C to 220°C.

8. The process of claim 6 or claim 7 wherein the process is conducted in bulk at a temperature of between 80°C and 200°C for from 3 to 300 minutes.

9. The process of any of claims 6 to 8 wherein the olefinic polymer is selected from butyl rubber and EPDM.

## Patentansprüche

1. Verbindung, die durch Umsetzen von allylischen Wasserstoff enthaltendem olefinischem Polymer mit einem Molekulargewicht von 500 bis 10⁷ ausgewählt aus Polymeren mit der Formel 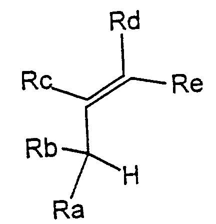 oder Ethylen, Propylen und nicht-konjugiertes Dienmonomer enthaltendem EPDM-Terpolymer, wobei Ra, Rb, Rc, Rd, Re unabhängig ausgewählt sind aus H, Alkylgruppen und substituierten Alkylgruppen mit 1 bis 10⁶ Kohlenstoffatomen, Alkenylgruppen und substituierten Alkenylgruppen mit 3 bis 10⁶ Kohlenstoffatomen, wobei die Substituenten ausgewählt sind aus Alkoxy, Halogen, CN, OH, HO (CH₂CH₂O)ₓ, wobei x 1 bis 10 beträgt, Acyl, Acyloxy, Arylsubstituenten, und 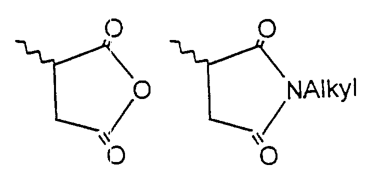 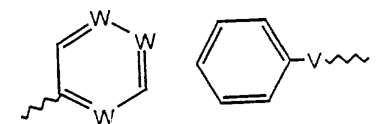 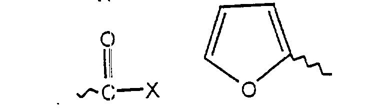 , wobei W=C, N; V=O, S, SO₂; und X ausgewählt ist aus der Gruppe bestehend aus -OH, -OR₁, -NR₁R₂, -R₁, wobei R₁ eine Gruppe mit 1 bis 18 Kohlenstoffatomen ist und R₂ Wasserstoff oder beliebiges Alkyl ist und 1 bis 18 Kohlenstoffatome aufweist,
mit acyclischer Verbindung ausgewählt aus
(A) acyclischem Carbonyl mit der Formel 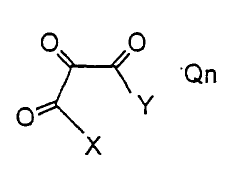 in der Q=HOH, MeOH, EtOH oder n-BuOH, n=0, 1, >1, und X und Y unabhängig ausgewählt sind aus -OH, -OR₁, -NR₁R₂, -R₁ und Phenyl, wobei R₁ eine Gruppe mit 1 bis 18 Kohlenstoffatomen ist und R₂ Wasserstoff oder eine Alkylgruppe mit 1 bis 18 Kohlenstoffatomen ist, und
(B) α-Ketobernsteinsäure, Diketobernsteinsäure, α-Ketokohlenwasserstoffsäuren und α,β-Diketokohlenwasserstoffsäuren, und den Ester- und Amidanaloga der α-Keto- und α,β-Diketokohlenwasserstoffsäuren mit der Maßgabe gebildet wird, dass das Molekulargewicht der α-Keto- und α,β-Diketokohlenwasserstoffsäuren und deren Ester- und Amidanaloga zwischen 130 und 500 beträgt.

2. Verbindung nach Anspruch 1, die durch Reaktion in Lösungsmittel bei Temperaturen von 50°C bis 220°C hergestellt ist.

3. Verbindung nach Anspruch 1 oder 2, die durch Reaktion in Masse bei Temperaturen von 80°C bis 220°C für 3 bis 300 Minuten hergestellt ist.

4. Verbindung nach Anspruch 2 oder 3, die durch Reaktion in Gegenwart von Säurekatalysator hergestellt worden ist.

5. Verbindung mit der Formel 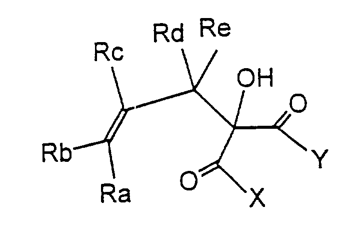 in der Ra, Rb, Rc, Rd, Re unabhängig ausgewählt sind aus H, Alkylgruppen und substituierten Alkylgruppen mit 1 bis 10⁶ Kohlenstoffatomen, Alkenylgruppen und substituierten Alkenylgruppen mit 3 bis 10⁶ Kohlenstoffatomen, wobei die Substituenten an den Alkyl- und/oder Alkenylgruppen ausgewählt sind aus Alkoxy, Halogen, CN, OH, HO(CH₂CH₂O)ₓ (wobei x 1 bis 10 beträgt), Acyl, Acyloxy, Arylgruppen, und 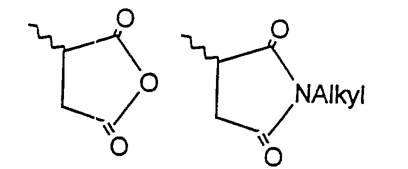 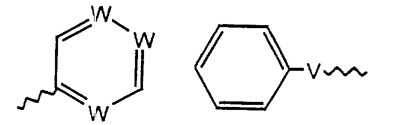 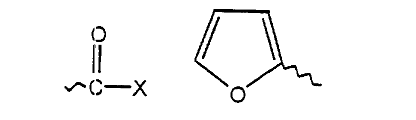 , wobei W=C, N; V=O, S, SO₂; und X ausgewählt ist aus der Gruppe bestehend aus -OH, -OR₁, -NR₁R₂, -R₁, wobei R₁ eine Gruppe mit 1 bis 18 Kohlenstoffatomen ist und R₂ Wasserstoff oder beliebiges Alkyl mit 1 bis 18 Kohlenstoffatomen ist, X und Y unabhängig ausgewählt sind aus -OH, -OR₁, -NR₁R₂, -R₁ und Phenyl, wobei R₁ eine Gruppe mit 1 bis 18 Kohlenstoffatomen ist und R₂ H oder eine Alkylgruppe mit 1 bis 18 Kohlenstoffatomen ist,
und wobei der Teil der Formel, welcher Ra, Rb, Rc, Rd und Re einschließt, ein Molekulargewicht von 500 bis 10⁷ aufweist.

6. Verfahren zur Herstellung von carbonylhaltiger Verbindung gemäß Anspruch 1 oder 5, bei dem das allylischen Wasserstoff enthaltende ungesättigte Kohlenwasserstoffpolymer mit einem Molekulargewicht von 500 bis 10⁷ mit der acyclischen Carbonylverbindung in Abwesenheit von freiradikalischen Initiatoren umgesetzt wird.

7. Verfahren nach Anspruch 6, das in Lösung bei Temperaturen von 50°C bis 220°C durchgeführt wird.

8. Verfahren nach Anspruch 6 oder Anspruch 7, das in Masse bei einer Temperatur von 80°C bis 200°C für 3 bis 300 Minuten durchgeführt wird.

9. Verfahren nach einem der Ansprüche 6 bis 8, bei dem das olefinische Polymer ausgewählt ist aus Butylkautschuk und EPDM.

## Revendications

1. Composé formé en faisant réagir un polymère oléfinique contenant un atome d'hydrogène allylique et ayant un poids moléculaire de 500 à 10⁷, choisi entre des polymères répondant à la formule : 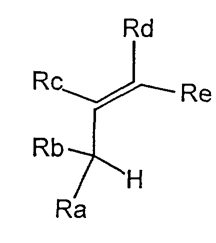 et un terpolymère EPDM contenant de l'éthylène, du propylène et un monomère diénique non conjugué, formule dans laquelle Ra, Rb, Rc, Rd et Re sont choisis, indépendamment, entre H, des groupes alkyle et des groupes alkyle substitués ayant 1 à 10⁶ atomes de carbone, des groupes alcényle et des groupes alcényle substitués ayant 3 à 10⁶ atomes de carbone, dans lesquels les substituants sont choisis entre des substituants alkoxy, halogéno, CN, OH, HO(CH₂CH₂O)ₓ, dans lequel x a une valeur de 1 à 10, acyle, acyloxy, aryle et 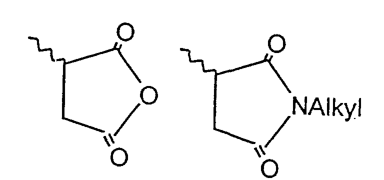 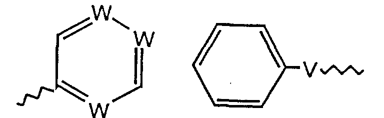 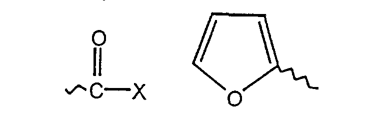 dans lesquels W représente C ou N ; V représente O, S ou un groupe SO₂ ; et X est choisi dans le groupe consistant en des groupes -OH ; -OR₁ ; -NR₁R₂ ; -R₁ ; dans lesquels R₁ représente un groupe ayant 1 à 18 atomes de carbone et R₂ représente l'hydrogène ou n'importe quel groupe alkyle ayant 1 à 18 atomes de carbone,
avec un composé acyclique choisi entre
(A) un composé carbonylé acyclique répondant à la formule : 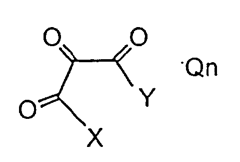 dans laquelle Q représente HOH, MeOH, EtOH ou n-BuOH ; n est égal à 0 ou 1 ou bien est supérieur à 1, et X et Y sont choisis, indépendamment, entre des groupes -OH, -OR₁, -NR₁R₂, -R₁ et phényle dans lesquels R₁ représente un groupe ayant 1 à 18 atomes de carbone et R₂ représente l'hydrogène ou un groupe alkyle ayant 1 à 18 atomes de carbone, et
(B) un acide alpha-cétosuccinique, un acide dicétosuccinique, des acides alpha-cétohydrocarboïques et des acides alpha,bêta-dicétohydrocarboïques, et les analogues consistant en esters et amides des acides alpha-céto- et alpha,bêta-dicéto-hydrocarboïques, sous réserve que le poids moléculaire des acides alpha-céto- et alpha,bêta-dicéto-carboïques et de leurs analogues consistant en esters et amides soit compris dans l'intervalle de 130 à 500.

2. Composé suivant la revendication 1, qui est produit par réaction dans un solvant à des températures comprises dans l'intervalle de 50°C à 220°C.

3. Composé suivant la revendication 1 ou 2, qui est produit par réaction en masse à des températures comprises dans l'intervalle de 80°C à 220°C pendant un temps de 3 à 300 minutes.

4. Composé suivant la revendication 2 ou 3, qui est produit par réaction en présence d'un catalyseur acide.

5. Composé répondant à la formule : 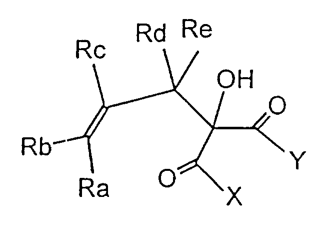 dans laquelle Ra, Rb, Rc, Rd et Re sont choisis, indépendamment, entre H, des groupes alkyle et des groupes alkyle substitués ayant 1 à 10⁶ atomes de carbone, les groupes alcényle et des groupes alcényle substitués ayant 3 à 10⁶ atomes de carbone, dans lesquels les substituants sur les groupes alkyle et/ou alcényle sont choisis entre des substituants alkoxy, halogéno, CN, OH, HO(CH₂CH₂O)ₓ (dans lequel x a une valeur de 1 à 10), acyle, acyloxy, aryle et 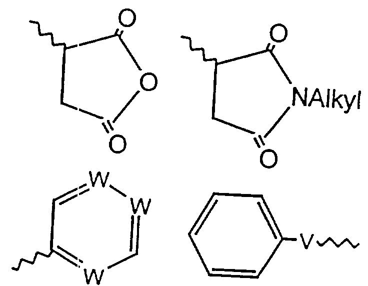 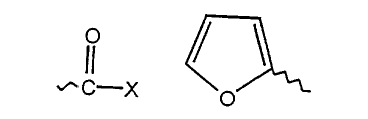 dans lesquels W représente C ou N ; V représente O, S ou un groupe SO₂ ; et X est choisi dans le groupe consistant en des groupes -OH ; -OR₁, -NR₁R₂ ; R₁ ; dans lesquels R₁ représente un groupe ayant 1 à 18 atomes de carbone et R₂ représente l'hydrogène ou n'importe quel groupe alkyle ayant 1 à 18 atomes de carbone,
X et Y sont choisis, indépendamment, entre des groupes -OH, -OR₁, -NR₁R₂, -R₁ et phényle dans lesquels R₁ représente un groupe ayant 1 à 18 atomes de carbone et R₂ représente H ou un groupe alkyle ayant 1 à 18 atomes de carbone,
et la portion de la formule comprenant Ra, Rb, Rc, Rd et Re ayant un poids moléculaire de 500 à 10⁷.

6. Procédé pour la production d'un composé à fonction carbonyle suivant la revendication 1 ou 5, comprenant la réaction du polymère hydrocarboné insaturé contenant un atome d'hydrogène allylique ayant un poids moléculaire de 500 à 10⁷ avec le composé carbonylé acyclique en l'absence d'initiateurs radicalaires.

7. Procédé suivant la revendication 6, qui est mis en oeuvre en solution à des températures comprises dans l'intervalle de 50°C à 220°C.

8. Procédé suivant la revendication 6 ou 7, qui est mis en oeuvre en masse à une température comprise dans l'intervalle de 80°C à 200°C pendant un temps de 3 à 300 minutes.

9. Procédé suivant l'une quelconque des revendications 6 à 8, dans lequel le polymère oléfinique est choisi entre le caoutchouc butyle et l'EPDM.
